# EUROPÄISCHE PATENTANMELDUNG

(11) **EP 2 018 833 A1**
(43) Veröffentlichungstag der Anmeldung: **28.01.2009**
(21) Anmeldenummer: 08012730.1
(22) Anmeldetag: 15.07.2008
(51) Int. Cl.: A61F 2/44, A61B 17/70

(54) **Wirbelsäulenimplantat**

(30) Priorität: 27.07.2007 DE 102007035667
(71) Anmelder: Unger, Michael, 78573 Wurmlingen (DE)
(72) Erfinder: Unger, Michael, 78573 Wurmlingen (DE)
(74) Vertreter: Weiss, Peter

(57) **Zusammenfassung**

Bei einem Wirbelsäulenimplantat, insbesondere zur Anbringung in Zwischenräume (4) von benachbarten Wirbelkörpern (1, 2), mit Stützflächen (5, 6, 33, 35) zur jeweiligen Anlage an die Begrenzungen der Zwischenräume (4), wobei zwischen den Stützflächen mindestens ein entsprechend dem Abstand der Stützflächen (5, 6, 33 35) veränderbares Ausgleichselement (7, 20 bis 23) vorgesehen ist, soll das Ausgleichselement (7; 20 - 23) des Wirbelsäulenimplantats (3 bzw. 3') in dessen implantiertem Zustand in mehr als zwei Freiheitsgraden bewegbar ausgebildet sein.

## Beschreibung

Die Erfindung betrifft ein Wirbelsäulenimplantat, insbesondere zur Anbringung in Zwischenräume von benachbarten Wirbelkörpern, mit Stützflächen zur jeweiligen Anlage an die Begrenzungen der Zwischenräume, wobei zwischen den Stützflächen mindestens ein entsprechend dem Abstand der Stützflächen veränderbares Ausgleichselement vorgesehen ist.

### Stand der Technik

Die Wirbelsäule besteht bekanntlich aus vielen einzelnen Wirbelkörpern, welche beweglich miteinander zu einem tragenden Skelettabschnitt verbunden sind. Damit die einzelnen Wirbelkörper die Einheit der Wirbelsäule bilden können, müssen sie stabil und gleichzeitig auch beweglich miteinander verbunden sein. Eine Verbindung, die diesen komplizierten Anforderungen entspricht, besteht dabei aus mehreren Komponenten. Neben kleinen Wirbelgelenken zwischen zwei benachbarten Wirbeln, die diese gelenkig miteinander verbinden und so eine gewisse Beweglichkeit gewährleisten, geben vor allem die sogenannten Bandscheiben zwischen benachbarten Wirbelkörpern sowie Bänder und Muskeln der Wirbelsäule Stabilität, aber gleichzeitig ermöglichen diese ebenfalls eine Bewegung.

Insbesondere intakte Bandscheiben sind in der Lage, Stosskräfte sanft abzufedern und ermöglichen gemeinsam mit den Wirbelgelenken Dreh-, Streck- und Beugebewegungen des menschlichen Rumpfes. Wenn nun in der Regel durch Verschleiss oder krankheitsbedingte Umstände sich z. B. einzelne Abstände der Wirbelkörper verringern, so kann dies zu schmerzhaften und bewegungseingeschränkten Krankheitsbildern führen.

Um hier Abhilfe zu schaffen, ist es bereits bekannt, Wirbelsäulenimplantate quasi als Abstandshalter zwischen den nicht intakten Wirbelkörpern vorzusehen. So ist hierfür ein Implantat bekannt, welches benachbarte Wirbelkörper in einem bestimmten Abstand zueinander halten soll, und zwar besitzt dieses Implantat dazu flanschartige Stützflächen zur jeweiligen Anlage an die zu beabstandenden Wirbelkörper, wobei diese flanschartigen Stützflächen einerseits über Befestigungsflansche an den benachbarten Wirbelkörpern durch Schrauben befestigt und andererseits auf der Seite des Wirbelzentrums über ein Bogenglied im Abstand zueinander gehalten werden. Durch die so erhaltene U-förmige Ausbildung, bei der das Bogenglied quasi als eine Art Ausgleichselement wirkt, soll dieses Implantat bei einer Rückenneigung komprimierbar sein, d. h., der Abstand der beiden Schenkel des U-förmigen Gebildes soll sich je nach Rückenbeugung nach hinten oder nach vorne verändern können, sodass dieses bekannte Wirbelsäulenimplantat maximal zwei Freiheitsgrade für die Bewegung der Wirbelsäule in diesem Bereich zulässt, was jedoch den Patienten dennoch in seinen Bewegungswünschen relativ stark einengt.

### Aufgabe

Hier setzt die vorliegende Erfindung ein, der die Aufgabe zugrunde liegt, ein Wirbelsäulenimplantat zu schaffen, mit dem ein Patient in Bezug auf eine bestimmte Abstandhaltung nicht intakter Wirbelkörper wieder eine verbesserte Lebensqualität hinsichtlich Schmerzen und allseitige Beweglichkeit wie Dreh-, Streck- und Beugebewegungen erhalten kann.

### Lösung der Aufgabe

Diese Aufgabe wird erfindungsgemäss dadurch gelöst, dass das Ausgleichselement des Wirbelsäulenimplantats in dessen implantiertem Zustand in mehr als zwei Freiheitsgraden bewegbar ausgebildet ist.

Dadurch werden die möglichen Bewegungsabläufe einer gesunden Wirbelsäule praktisch weitgehend wieder hergestellt.

Gemäss einer bevorzugten Ausgestaltung der Erfindung ist es von Vorteil, wenn als Ausgleichselement ein geschlossener Hohlkörper dient, der mit seinen Stirnseiten mit den Stützflächen des Implantates verbunden ist und eine balgartige äussere Begrenzungsfläche aufweist. Nach Massgabe dieser Ausgestaltung ist es dabei zweckmässig, dass die balgartige Ausführung des als Ausgleichselement dienenden Hohlkörpers derart beschaffen ist, dass sich dieser in Richtung seiner Mittelachse und winkelabweichend von dieser elastisch verhält, wobei das elastische Verhalten nach Art einer steifen Spiralfeder bestimmt werden kann.

Damit erlaubt ein derart ausgebildetes Wirbelsäulenimplantat eine.Vielzahl von Bewegungen der Wirbelsäule, was einer Mehrzahl über zwei an Freiheitsgraden entspricht, und zwar indem sich der Abstand zum einen in Richtung der Mittelachse des Ausgleichselements vergrössern oder verkleinern kann, was für die Aufgabe, als eine Art Stossdämpfer zu dienen, sehr wichtig ist, z. B. bei einem Sturz auf das Gesäss. Zum anderen sind von der Mittelachse winkelabweichende Bewegungen möglich, wie ein Beugen nach vorne, hinten und seitlich, sodass mit dem erfindungsgemässen Wirbelsäulenimplantat ein weitgehend normaler Bewegungsablauf der Wirbelsäule praktisch wie in einem gesunden Zustand gegeben ist.

Die äussere Form des Hohlkörpers des Ausgleichselements kann in seiner Transversalebene zylindrisch, oval, nierenförmig oder dgl. ausgebildet sein, wobei bei einer Verwendung des erfindungsgemässen Wirbelsäulenimplantats als Ersatzbandscheibe der Hohlkörper des Ausgleichselements eine Höhe von ca. 4 - 9 mm und einen äusseren Durchmesser von ca. 14 - 16 mm besitzen kann.

Zur Fixierung des erfindungsgemässen Wirbelsäulenimplantats an der Wirbelsäule ist es zweckmässig, dass an diesem Befestigungsflansche vorgesehen sind, mit denen das Implantat an benachbarte Wirbelkörper anschraubbar ist, wobei es sich bewährt hat, dass die Befestigungsflansche mindestens zwei Bohrungen aufweisen, die in einer Transversalebene der Wirbelkörper verlaufen.

Bei der bisher beschriebenen Ausführungsform ist der als Ausgleichselement dienende Hohlkörper mit den zugehörigen Stützflächen des Wirbelsäulenimplantats fest verbunden, sodass dieses bei einer möglichen Drehung des Rumpfes gewissen Torsionskräften ausgesetzt ist, was jedoch durch die Elastizität des Ausgleichselements in der Regel nicht schadet.

Um nun eine weitere Erhöhung der angestrebten Bewegungs-Freiheitsgrade zu erreichen, kann es ferner zweckmässig sein, dass der als Ausgleichselement dienende Hohlkörper mit seiner einen Stirnseite fest und mit seiner anderen Stirnseite drehbeweglich mit der jeweiligen Stützfläche des Implantats verbunden ist. In einem solchen Fall, kann sich dann der Patient problemlos auch noch seitlich nach rechts und links drehen. Bei einer derart angestrebten Drehbewegung ist es von Vorteil, wenn die drehbewegliche Seite des als Hohlkörper ausgebildeten Ausgleichselements durch einen an der zugehörigen Stützfläche vorgesehenen Zapfen drehbeweglich geführt ist, wobei als Regelausführung sowohl der Hohlkörper als auch der Zapfen dabei eine zylindrische Form haben können.

Bei dieser Konstruktion ist es auch möglich, dass die Drehbeweglichkeit begrenzt wird, und zwar indem man eine von der zylindrischen Form abweichende Aussenform des als Hohlkörper ausgebildeten Ausgleichselements wählt, wobei der an der zugehörigen Stützfläche vorgesehene Zapfen die gleiche Aussenkontur wie der lichte Innenquerschnitt des Hohlkörpers erhält. Dabei ist jedoch der Zapfen von seiner Querschnittsausdehnung her gesehen entsprechend der Grösse eines gewünschten Ausschlagswinkels einer zugehörigen Drehbewegung kleiner als der lichte Innenquerschnitt des Hohlkörpers ausgebildet, um einen gewissen Bewegungsspielraum zu erhalten. Deshalb ist hierfür eine ovale Form für den Hohlkörper und den Zapfen bestens geeignet, und zwar kann sich dann der ovale Zapfen innerhalb des grösseren Ovals des Hohlkörpers wie ein Art Kulissenstein begrenzt bewegen.

Bei einem Einsatz des erfindungsgemässen Wirbelsäulenimplantats als Bandscheibenersatz ist es zweckmässig, dass die Stützflächen zur jeweiligen Anlage an die benachbarten Begrenzungen der Zwischenräume in ihrer jeweiligen flächigen Ausdehnung etwa entsprechend der jeweiligen Grösse der durch die Wirbelkörper bestimmten Begrenzungen ausgebildet sind. Dies ist auch deshalb angezeigt, da so die Druckbelastung praktisch auf die gesamte Wirbelkörperoberfläche abgeleitet werden kann.

Die jeweiligen Stützflächen sind zweckmässigerweise durch Flansche gebildet, deren Dicken auch an die vorgegebenen Einbauverhältnisse angepasst sein können. So kann ihre Dicke auch entsprechend der Winkellage der benachbarten Begrenzungen der Zwischenräume keilartig ausgebildet sein, da sich in der Regel der Zwischenraum zwischen zwei benachbarten Wirbelkörpern im Bereich der Bandscheibe vom Zentrum einer Wirbelsäule aus nach aussen vergrössert.

Das erfindungsgemässe Wirbelsäulenimplantat erlaubt aber auch, dass es mit einem Wirbelersatzkörper kombiniert wird, der dann entweder einstückig mit der zugehörigen flanschartigen Stützfläche ausgebildet oder mit dieser fest oder lösbar verbunden wird, wobei ein derartiger Wirbelersatzkörper zweckmässigerweise eine Dicke von ca. 12 - 28 mm aufweisen kann.

Schliesslich ist es von Vorteil, wenn das erfindungsgemässe Wirbelsäulenimplantat aus einer körperverträglichen Titanlegierung gefertigt ist.

### Figurenbeschreibung

Weitere Merkmale und Vorteile der vorliegenden Erfindung ergeben sich aus der nachfolgenden Beschreibung mehrerer Ausführungsbeispiele in Verbindung mit der Zeichnung; es zeigen
- Fig. 1: eine vereinfachte Darstellung von zwei benachbarten Wirbelkörpern in Seitenansicht, zwischen denen ein Wirbelsäulenimplantat implantiert ist, dessen balgartiges Ausgleichselement zylindrisch oder oval sein kann;
- Fig. 2: eine weiter vereinfachte Darstellung der zwei benachbarten Wirbelkörper gemäss Fig. 1, von der Seite gemäss der Linie II - II in Fig. 1 gesehen;
- Fig. 3: eine Draufsicht auf einen vereinfacht dargestellten Lendenwirbel mit zwei angedeuteten, im Querschnitt zylindrischen Ausgleichselementen und wahlweise einem angedeuteten, im Querschnitt ovalen Ausgleichselement;
- Fig. 4: eine vereinfachte Darstellung von zwei benachbarten Wirbelkörpern gemäss Fig. 1 mit einem Ausgleichselement, das mittels einer zylindrischen Zapfenverbindung drehbeweglich ausgebildet ist;
- Fig. 5: eine Prinzipsskizze in vergrösserter Darstellung als Schnitt durch ein im Querschnitt ovales Ausgleichselement mit einem ebenfalls im Querschnitt ovalen Drehzapfen, wobei beide Querschnitte für begrenzte Dreh- und Schubbewegungen unterschiedlich gross ausgebildet sind;
- Fig. 6: eine Draufsicht auf die Prinzipsskizze gemäss Fig. 5 mit zwei Lagedarstellungen des ovalen Drehzapfens;
- Fig. 7: einen vereinfacht dargestellten Ausschnitt als Seitenansicht von zwei benachbarten Wirbelkörpern mit zwei Korrekturbeispielen hinsichtlich ihrer Lage zueinander und ihrer Wirbelbeschaffenheit.

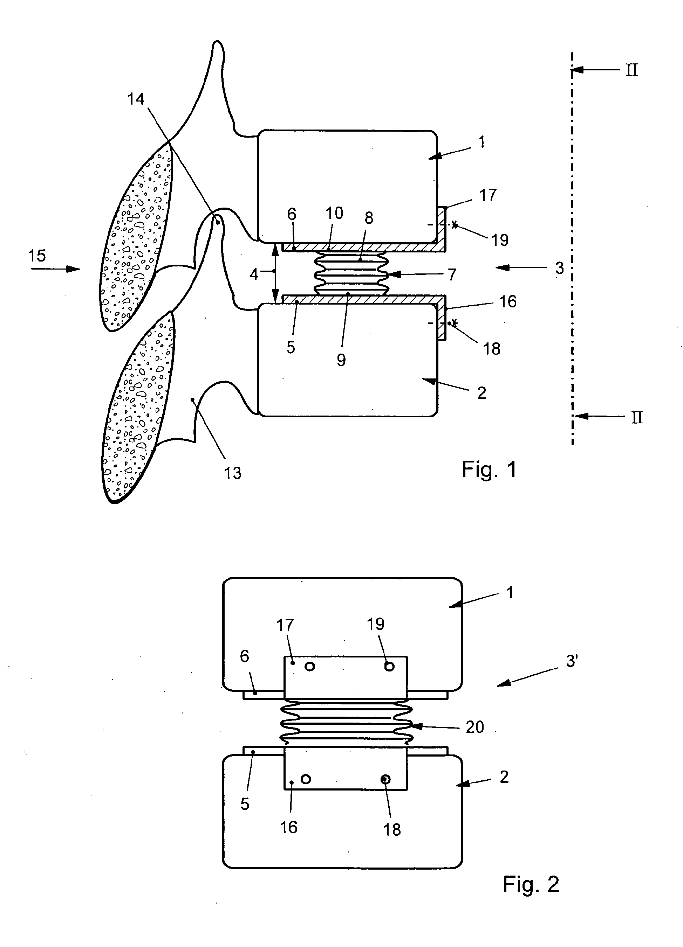

Fig. 1 zeigt in einer vereinfacht dargestellten Seitenansicht zwei benachbarte Wirbelkörper 1 und 2, zwischen denen ein insgesamt mit 3 bezeichnetes Wirbelsäulenimplantat aus einer körperverträglichen Titanlegierung implantiert ist. Bei diesem Ausführungsbeispiel wird davon ausgegangen, dass ein Bandscheibenvorfall vorlag und die im Zwischenraum - bezeichnet mit 4 - zwischen den Wirbelkörpern 1 und 2 vorhanden gewesene Bandscheibe ganz entfernt werden musste. Das nun an Stelle der Bandscheibe implantierte Wirbelsäulenimplantat 3 übernimmt nunmehr die Aufgabe als Distanzhalter, wozu auch die Bezeichnung "cage" verwendet wird. Mit einem derartigen Distanzhalter kann einerseits der Abstand zwischen den Wirbelflächen der benachbarten Wirbelkörper 1 und 2 erhalten und andererseits können diese miteinander verbunden werden.

Wie in Fig. 1 gezeigt, kann die Verbindung der beiden Wirbelkörper 1 und 2 so erfolgen, dass es sich um eine feste Verbindung handelt. Hierzu besitzt das Wirbelsäulenimplantat 3 zwei sich gegenüberstehende flanschartige Stützflächen 5 und 6, zwischen denen ein balgartig ausgebildetes Ausgleichselement 7 angeordnet ist. Das Ausgleichselement 7 besteht aus einem geschlossenen Hohlkörper 8 und ist an seinen beiden Stirnseiten 9 und 10 mit den jeweiligen flanschartigen Stützflächen 5 und 6 fest verbunden.

Durch die balgartige Ausführung des Aussenmantels des Hohlkörpers 8, ist die Verbindung zwischen den Wirbelkörpern 1 und 2 dennoch elastisch, d. h., das Ausgleichselement 7 lässt dadurch begrenzte Bewegungen einmal in beide Richtungen seiner Mittelachse und von dieser winkelabweichend zu. Somit ergeben sich dadurch vorteilhaft zwei Bewegungsfreiheitsgrade nach oben und nach unten sowie eine Vielzahl davon in diverse Richtungen von Beugungsbewegungen. Das elastische Verhalten des Ausgleichelements 7 wurde dabei bei seiner Herstellung nach der Art einer steifen Spiralfeder bestimmt.

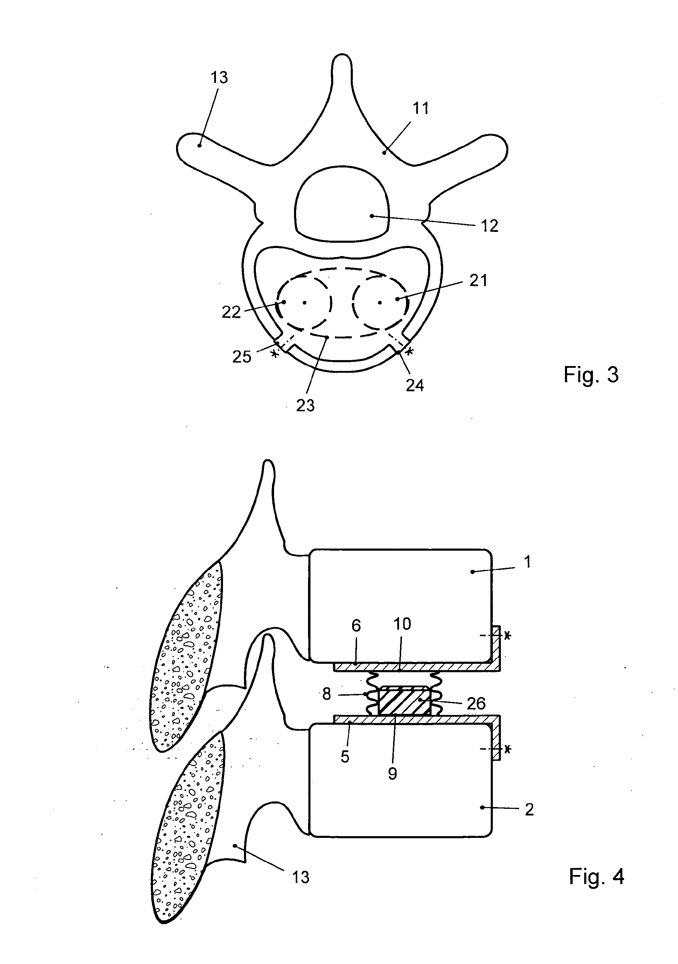

Die Wirbelkörper 1 und 2 besitzen darüber hinaus jeweils einen sogenannten Wirbelbogen 11 (s. Fig. 3)., der das sogenannte Wirbelloch - bezeichnet mit 12 - umgibt und Fortsätze 13 aufweist. Ferner sind die einzelnen Wirbel einer Wirbelsäule jeweils mit einem sogenannten Wirbelgelenk 14 (s. Fig. 1) miteinander verbunden, wobei die Wirbelkörper 1 und 2 ins Körperinnere des Menschen und die Fortsätze 13 in Richtung seines Rückens gerichtet sind, was durch den Pfeil 15 in Fig. 1 angedeutet werden soll.

In diesem Zusammenhang wird hervorgehoben, dass das erfindungsgemässe Wirbelsäulenimplantat nicht nur zwischen den einzelnen Wirbelkörpern als solche als Distanzhalter eingesetzte werden kann, sondern auch zwischen den Dornfortsätzen 13, wodurch die Wirbelgelenke 14 nachhaltig entlastet werden können, um z. B. Schmerzen durch mögliche eingeklemmte Nerven zu verhindern.

In Fig. 2 ist die Darstellung der zwei benachbarten Wirbelkörper 1 und 2 von der Seite gemäss der Linie II - II in Fig. 1 zu sehen, wobei hier die Fortsätze 13 weggelassen wurden. Wie auch in Fig. 1 dargestellt, besitzen die flanschartigen Stützflächen 5 und 6 Befestigungsflansche 16 und 17, mit denen das Wirbelsäulenimplantat 3 bzw. 3 an den Wirbelkörpern 1 und 2 mechanisch mittels Schrauben 18 und 19 befestigt sind, deren Schraubenbohrungen jeweils in einer Transversalebene der Wirbelkörper 1 und 2 verlaufen.

Bei dem Wirbelsäulenimplantat 3' handelt es sich um eine weitere Ausführungsform eines Ausgleichelements 20 mit einer ovalen Querschnittsform, von der in Fig: 2 die Breitseite zu sehen ist und in Fig: 1 als weiteres Ausführungsbeispiel die Schmalseite angenommen werden kann. Mit einer derart ovalen Querschnittsform, welche auch nierenförmig oder dgl. sein kann, wird eine breitere Lasttragung erreicht.

Der Hohlkörper 8 des Wirbelsäulenimplantats 3, hat im vorliegenden Ausführungsbeispiel eine Höhe von 6 mm, wobei diese auch zwischen ca. 4 - 9 mm gewählt werden kann, und bei einer zylinderischer Ausführung einen Durchmesser von 15 mm, der jedoch auch 14 oder 16 mm sein kann. Bei einer ovalen Ausführung, wie es bei dem Ausgleichsmoment 20 des Wirbelsäulenimplantats 3' der Fall ist, sind dann dessen Hauptachse im Rahmen dieser Masse grösser als die Achse der Schmalseite gewählt.

Im Lendenwirbelbereich einer Wirbelsäule, wo die Wirbelscheiben z. B. gegenüber denen im Brustwirbelbereich grösser sind, kann es auch zweckmässig sein, zwei als Hohlkörper ausgebildete Ausgleichselemente 21 und 22 oder ein grösseres im Querschnitt ovalförmiges Ausgleichselement 23 zu verwenden. Letztere sind alternativ in Fig. 3 angedeutet, wobei dann jede der zugehörigen flanschartigen Stützflächen, von denen eine in Fig. 3 zu sehen ist, zweckmässigerweise zwei in einem gewissen Abstand voneinander angeordnete Befestigungsflansche 24 und 25 besitzen kann.

In Fig. 4 ist eine weitere Ausführungsform gezeigt, bei der die zwei benachbarten Wirbelkörper 1 und 2 drehbeweglich miteinander verbunden sind. Hierbei ist der Hohlkörper 8 mit seiner oberen Stirnseite 10 fest mit der oberen flanschartigen Stützfläche 6 verbunden, wogegen seine untere Stirnseite 9 gegenüber der unteren flanschartigen Stützfläche 5 frei beweglich ist, d. h., sie ist nicht an dieser befestigt. Von der unteren Stützfläche 5 aus ragt dagegen ein zylindrischer Zapfen 26 in den Innenraum des Hohlkörpers 8, der diesen drehbeweglich führt und somit vorteilhaft einen weiteren Freiheitsgrad, nämlich Drehbewegungen, dieser Implantatverbindung zulässt, wobei die Anordnung des Drehzapfens 26 auch umgekehrt sein kann, d. h., an der oberen Stützfläche 6.

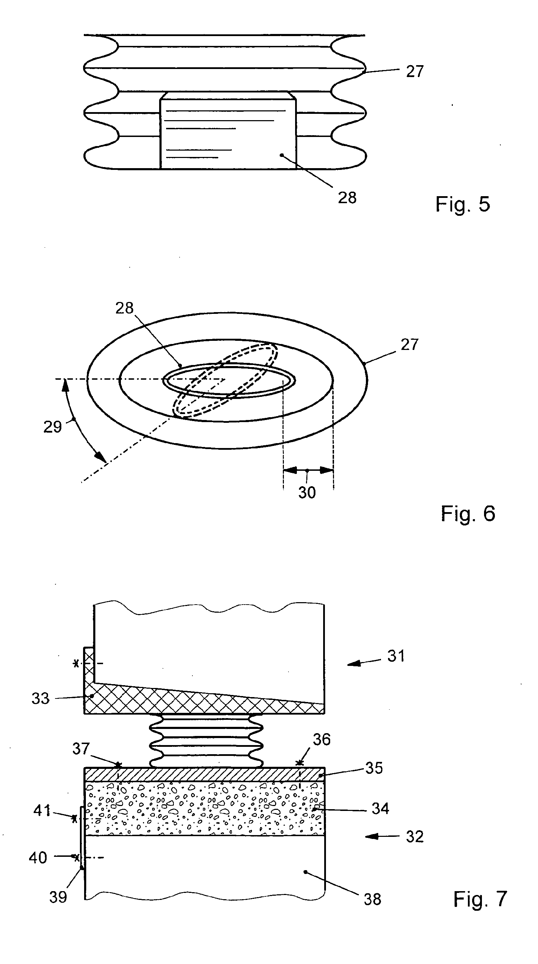

Falls eine derartige offene Drehbeweglichkeit z. B. aus medizinischen Gründen nicht erwünscht ist, kann diese auch begrenzt werden, was in Fig. 5 und 6 durch Prinzipsskizzen dargestellt ist. Gemäss Fig. 5 ist hierfür ein als Ausgleichselement dienender, balgartiger Hohlkörper 27 vorgesehen, der einen ovalen Querschnitt besitzt. Entsprechend der Ausführungsform in Fig. 4 greift auch hier ein Drehzapfen 28 in den Innenraum des Hohlkörpeers 27 ein, welcher die gleiche ovale Aussenkontur wie der lichte Innenquerschnitt des Hohlkörpers 27 besitzt. Der Drehzapfen 28 ist jedoch von seiner Querschnittsausdehnung her gesehen entsprechend der Grösse eines gewünschten Ausschlagswinkels einer zugehörigen Drehbewegung - bezeichnet mit 29 - kleiner als der lichte Innenquerschnitt des Hohlkörpers 27 ausgebildet.

Wie aus Fig. 6 ersichtlich, die eine Draufsicht auf den Hohlkörper 27 mit Zapfen 28 darstellt, wird die Grösse des Ausschlagswinkels 29 und somit die zugehörige mögliche Drehbewegung des Drehbolzens 28 durch die Länge der Grossachse seiner Ovalform bestimmt. Gleichzeitig ist dadurch auch eine relatives Verschieben des Drehzapfens 28 innerhalb des Hohlkörpers 27 bzw. des Innenraums des Hohlkörpers 27 zu dem Drehzapfen 28 gegeben, was mit dem Doppelpfeil 30 ausgedrückt ist. Hierdurch erhält ein derartig ausgebildetes Wirbelsäulenimplantat vorteilhaft einen weiteren Freiheitsgrad hinsichtlich einer damit erreichbaren Bewegungsfreiheit. In Fig. 6 sind hierzu zwei Lagen des Drehzapfens 28 gezeigt; einmal vollausgezogen in Ausgangsstellung und einmal gestrichelt in maximal Schwenkposition.

Schliesslich sind in Fig. 7 zwei Beispiele einer möglichen Korrektur hinsichtlich einer unterschiedlichen Beschaffenheit vorhandener Wirbelkörper 31 und 32 angegeben, und zwar im Zusammenhang mit einem erfindungsgemässen Wirbelsäulenimplantat. Im Fälle einer Schieflage des Wirbelkörpers 31 kann diese z. B. durch eine entsprechend keilartige Ausbildung der dazugehörigen flanschartigen Stützfläche 33 erfolgen. Oder im Falle, dass ein Wirbelersatzkörper erforderlich ist, kann dieser - bezeichnet mit 34 - unmittelbar mit der unteren flanschartigen Stützfläche - bezeichnet mit 35 - verbunden werden. Hierbei ist es möglich, dass der Wirbelsatzkörper 34 einstückig mit der Stützfläche 35 ausgebildet oder aber mit dieser fest oder lösbar verbunden wird, was in Fig. 7 z. B. durch die als Pfeile dargestellten Schrauben 36 und 37 angedeutet ist. Auch ist es möglich, den Wirbelersatzkörper z. B. mit dem Rest eines noch vorhandenen Wirbelkörpers 38 zu verbinden, was durch einen Seitenflansch 39 mit Schrauben 40 und 41 erfolgen kann.

Insgesamt ist mit der vorliegenden Erfindung ein Zwischenwirbelimplantat in der Form eines elastischen Distanzhalters geschaffen worden, der aufgrund seiner Elastizität einerseits und seiner Vielzahl an zusätzlichen Freiheitsgraden andererseits dem Patienten trotz eines ursächlichen Wirbelsäulendefekts wieder eine "komfortable" Beweglichkeit gewährt, wie gute Beuge-, Streck- und Drehbewegungen.

Neben dem Einsatz des erfindungsgemässem Wirbelsäulenimplantats im Bereichen der Bandscheiben ist es darüber hinaus auch möglich, derartige Implantate als Distanzhalter zwischen den Dornfortsätzen an den Wirbelkörpern in geeigneter Weise zu implantieren, wodurch die Wirbelgelenke als solche nachhaltig entlastet werden können, um z. B. mögliche Schmerzen eines Patienten, hervorgerufen durch eingeklemmte Nerven, zu beseitigen bzw. zu verhindern.

### Bezugszeichenliste

| | | | | | |
|---|---|---|---|---|---|
| 1 | Wirbelkörper | 34 | Wirbelersatzkörper | 67 | |
| 2 | Wirbelkörper | 35 | Stützflansch | 68 | |
| 3; 3' | Wirbelsäulenimplantat | 36 | Schraube | 69 | |
| 4 | Zwischenraum | 37 | Schraube | 70 | |
| 5 | Stützfläche | 38 | Restwirbelkörper | 71 | |
| 6 | Stützfläche | 39 | Seitenflansch | 72 | |
| 7 | Ausgleichselement | 40 | Schraube | 73 | |
| 8 | Hohlkörper | 41 | Schraube | 74 | |
| 9 | Stirnseite | 42 | | 75 | |
| 10 | Stirnseite | 43 | | 76 | |
| 11 | Wirbelbogen | 44 | | 77 | |
| 12 | Wirbelloch | 45 | | 78 | |
| 13 | Fortsätze | 46 | | 79 | |
| 14 | Wirbelgelenk | 47 | | | |
| 15 | Peil (Rückenansicht) | 48 | | | |
| 16 | Befestigungsflansch | 49 | | | |
| 17 | Befestigungsflansch | 50 | | | |
| 18 | Schraube | 51 | | | |
| 19 | Schraube | 52 | | | |
| 20 | Ausgleichselement | 53 | | | |
| 21 | Ausgleichselement | 54 | | | |
| 22 | Ausgleichselement | 55 | | | |
| 23 | Ausgleichselement | 56 | | | |
| 24 | Befestigungsflansch | 57 | | | |
| 25 | Befestigungsflansch | 58 | | | |
| 26 | zylindrischer Zapfen | 59 | | | |
| 27 | ovaler Hohlkörper | 60 | | | |
| 28 | Drehzapfen | 61 | | | |
| 29 | Ausschlagwinkel | 62 | | | |
| 30 | Doppelpfeil | 63 | | | |
| 31 | Wirbelkörper | 64 | | | |
| 32 | Wirbelkörper | 65 | | | |
| 33 | keilf. Stützfläche | 66 | | | |

## Patentansprüche

1. Wirbelsäulenimplantat, insbesondere zur Anbringung in Zwischenräume (4) von benachbarten Wirbelkörpern (1, 2), mit Stützflächen (5, 6, 33, 35) zur jeweiligen Anlage an die Begrenzungen der Zwischenräume (4), wobei zwischen den Stützflächen mindestens ein entsprechend dem Abstand der Stützflächen (5, 6, 33 35) veränderbares Ausgleichselement (7, 20 bis 23) vorgesehen ist,
**dadurch gekennzeichnet,**
**dass** das Ausgleichselement (7; 20 - 23) des Wirbelsäulenimplantats (3 bzw. 3') in dessen implantiertem Zustand in mehr als zwei Freiheitsgraden bewegbar ausgebildet ist.

2. Wirbelsäulenimplantat nach Anspruch 1, **dadurch gekennzeichnet, dass** als Ausgleichselement (7; 20 - 23) ein geschlossener Hohlkörper (8, 27) dient, der mit seinen Stirnseiten (9, 10) mit den Stützflächen (5, 6) verbunden ist und eine balgartige äussere Begrenzungsfläche aufweist.

3. Wirbelsäulenimplantat nach Anspruch 2, **dadurch gekennzeichnet, dass** die balgartige Ausführung des als Ausgleichselement (7; 20 - 23) dienenden Hohlkörpers (8, 27) derart beschaffen ist, dass sich dieser in Richtung seiner Mittelachse und winkelabweichend von dieser elastisch verhält, wobei bevorzugt das elastische Verhalten des als Hohlkörper (8) ausgebildeten Ausgleichselements (7; 20 - 23) nach der Art einer steifen Spiralfeder bestimmt ist.

4. Wirbelsäulenimplantat nach wenigstens einem der Ansprüche 1 bis 3, **dadurch gekennzeichnet, dass** die äussere Form des Hohlkörpers (8) des Ausgleichelements (7; 20 - 23) in der Transversalebene zylindrisch, oval, nierenförmig oder dgl. ausgebildet ist.

5. Wirbelsäulenimplantat nach einem der vorhergehenden Ansprüche, **dadurch gekennzeichnet, dass** Befestigungsflansche (16, 17; 24, 25) vorgesehen sind, die an benachbarte Wirbelkörper (1, 2) anschraubbar sind, wobei sie mindestens zwei Bohrungen (18, 19) aufweisen, die in einer Transversalebene der Wirbelkörper (1, 2) verlaufen.

6. Wirbelsäulenimplantat nach einem der vorhergehenden Ansprüche, **dadurch gekennzeichnet, dass** der Hohlkörper (8) mit den zugehörigen Stützflächen (5, 6) fest verbunden ist.

7. Wirbelsäulenimplantat nach mindestens einem der Ansprüche 1 bis 5, **dadurch gekennzeichnet, dass** der Hohlkörper (27) mit den zugehörigen Stützflächen (5, 6) drehbeweglich verbunden ist.

8. Wirbelsäulenimplantat nach Anspruch 7, **dadurch gekennzeichnet, dass** der Hohlkörper (8 bzw. 27) mit seiner einen Stirnseite (10) fest und mit seiner anderen Stirnseite (9) drehbeweglich mit der jeweiligen Stützfläche (6, 5) verbunden ist.

9. Wirbelsäulenimplantat nach Anspruch 8, **dadurch gekennzeichnet, dass** an der drehbeweglichen Stirnseite (9) des als Hohlkörper (8 bzw. 27) ausgebildeten Ausgleichselement durch einen an der zugehörigen Stützfläche (9) vorgesehenen Zapfen (26 bzw. 28) drehbeweglich geführt ist.

10. Wirbelsäulenimplantat nach Anspruch 9, **dadurch gekennzeichnet, dass** die Drehbeweglichkeit des als Hohlkörper (27) ausgebildeten Ausgleichselement durch den an der zugehörigen Stützfläche (5) vorgesehenen Zapfen (28) begrenzt ist.

11. Wirbelsäulenimplantat nach Anspruch 10, **dadurch gekennzeichnet, dass** bei einer von der zylindrischen Form abweichenden Aussenkontur des als Hohlkörper (27) ausgebildeten Ausgleichelement die Begrenzung der Drehbeweglichkeit **dadurch** erreicht ist, dass der an der zugehörigen Stützfläche (5) vorgesehene Zapfen (28) zwar vorzugsweise die gleiche Aussenkontur wie der lichte Innenquerschnitt des Hohlkörpers (27) aufweist, jedoch von seiner Querschnittsausdehnung her entsprechend der Grösse eines gewünschten Ausschlagswinkels (29) einer zugehörigen Drehbewegung kleiner als der lichte Innenquerschnitt des Hohlkörpers (27) ausgebildet ist.

12. Wirbelsäulenimplantat nach wenigstens einem der vorhergehenden Ansprüche, **dadurch gekennzeichnet, dass** die jeweiligen flanschartigen Stützflächen (33) in ihren Dicken entsprechend der Winkellage der benachbarten Begrenzungen des Wirbelkörpers (31) keilartig ausgebildet sind.

13. Wirbelsäulenimplantat nach wenigstens einem der vorhergehenden Ansprüche, **dadurch gekennzeichnet, dass** mindestens eine flanschartige Stützfläche (35) zur Anlage an die benachbarte Begrenzung eines Zwischenraums mit einem Wirbelersatzkörper (34) ggf. einstückig versehen ist.

14. Wirbelsäulenimplantat nach einem der vorhergehenden Ansprüche, **dadurch gekennzeichnet, dass** dieses zwei Ausgleichselemente (21, 22) aufweist.

15. Wirbelsäulenimplantat nach einem der vorhergehenden Ansprüche, **dadurch gekennzeichnet, dass** dieses aus einer körperverträglichen Titanlegierung gefertigt ist.
